# EUROPEAN PATENT APPLICATION

(11) **EP 1 935 366 A1**
(43) Date of publication of application: **25.06.2008**
(21) Application number: 07255009.8
(22) Date of filing: 21.12.2007
(51) Int. Cl.: A61B 19/02

(54) **Instrument tracking container and method**

(30) Priority: 22.12.2006 US 615286
(71) Applicant: Ethicon, Inc, Somerville, NJ 08876-0151 (US)
(72) Inventor: Jackson, Richard A., Huntington Beach, CA 92649 (US)
(74) Representative: Tunstall, Christopher Stephen

(57) **Abstract**

A system and method provide for holding and tracking medical instruments (12). The method includes the steps of: associating one or more of the medical instruments (12) with a container (10) and storing that association within a control system associated with the container, each of the medical instruments bearing a machine readable identification tag (60) bearing information about itself; querying a contents of the container with a tag reader (62) to identify which instruments are therein; and determining whether the container contains each of the instruments associated with the container and only those instruments and outputting that determination to a user.

## Description

### Field of the Invention

This application relates to storing medical instruments in a container, and more specifically to insuring that the proper instruments are within the container.

### Background of the Invention

Medical instruments are commonly held within containers. These container can include sterilization trays and soaking containers. Especially in a container containing multiple instruments designed for a particular medical procedure, a procedure set, it is important to have all of the instruments necessary and desirable to return all of the reusable instruments to the container after a procedure is complete. This can be challenging and is typically accomplished with a sheet bearing a list of the instruments which is checked as they are loaded. Checking their return after a procedure is even more challenging due to the other concerns facing the personnel during the procedure.

### Summary of the Invention

The present invention overcomes these and other limitations in the prior art.

A system, according to the present invention, provides for holding and tracking medical instruments. The system comprises a container for holding at least one of the medical instruments associated with the container. Each of the medical instruments bears a machine readable identification tag bearing information about that instrument. A tag reader is associated with the container and a control system is linked to the tag reader which compares the information as read by the tag reader with pre-programmed data to match the at least one instrument to the container.

Preferably, the control system further includes an output device which indicates based upon the information read from the tag and the pre-programmed data whether the at least one instrument belongs in the container.

In one aspect of the invention, a plurality of the instruments are associated with the container and the control system is programmed to detect whether all of the instruments are properly within the container.
Preferably, the control system further includes an output device to inform a user whether all of the instruments are properly within the container, and when one or more of the instruments associated with the container are not in the container, the output device further lists which of the instruments are missing from the container. Also, preferably, if one or more instruments not associated with the container are detected in the container the output device further lists which instruments are improperly in the container.

In one aspect of the invention, each of the instruments is identified by its type. This could include broad types such as "forceps" or be more specific to include sized and particular types or shapes of forceps. Each of the instruments can also be, or alternatively be, identified with a unique identifier not shared by another instrument. This would be most useful with more complex instruments such as endoscopes.

Preferably, the tags are RFID tags and the tag reader is an RFID tag reader. The tag reader can be located on the container, or be separate, such as a small handheld device which can be held near the container.

A method, according to the present invention, provides for holding and tracking medical instruments. The method includes the steps of: associating one or more of the medical instruments with a container and storing that association within a control system associated with the container, each of the medical instruments bearing a machine readable identification tag bearing information about itself; querying a contents of the container with a tag reader to identify which instruments are therein; and determining whether the container contains each of the instruments associated with the container and only those instruments and outputting that determination to a user.

When a plurality of the instruments are associated with the container, the method preferably includes the step of informing the user whether all of the instruments are properly within the container, and further the step of when one or more of the instruments associated with the container are not in the container, informing the user which of the instruments are missing from the container. If one or more instruments not associated with the container are detected in the container the method preferably includes the step of informing the user which instruments are improperly in the container.

In one aspect of the invention the instruments comprise a set designed for a medical procedure and the method includes the step of making sure each of the instruments of the set are placed into the container prior to the procedure. Further, the method can include the step of ensuring that each of the instruments in the set which are reusable are returned to the container after the medical procedure. Some instruments in the set might be tagged for inclusion during pre-procedure loading, but be designed to be disposed of after the procedure and it is not necessary to track their return to the container.

### Brief Description of the Drawings

FIG. 1 is a front elevation view of a container of the present invention;
FIG. 2 is a block diagram of a communication system for use with the container of FIG. 1; and
FIG. 3 is a block diagram of a handheld device for use with the container of FIG. 1 and communication system of FIG 2.

### Detailed Description of the Invention

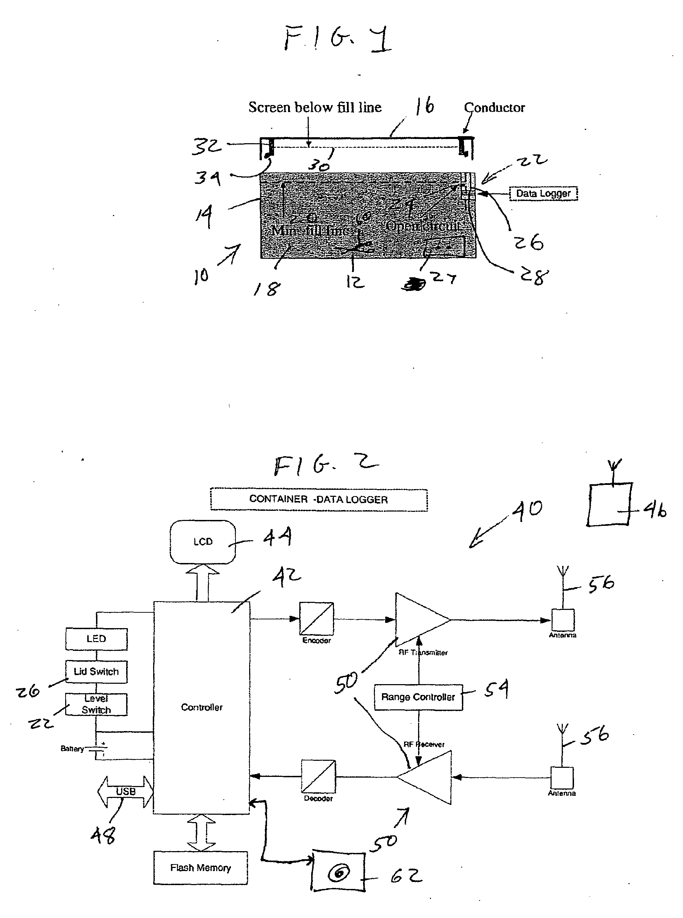

FIG. 1 discloses a container 10 adapted for soaking medical instruments 12 after their use in a medical procedure. It comprises a basin 14 and a lid 16 which fits upon the basin 14. The basin 14 is preferably fluid tight and sized to accommodate one or more of the surgical instruments 12 and to accommodate a soaking solution 18 up to a fill line 20. Preferably, the lid 16 is also fluid tight and tightly fitting such that when the lid 16 is placed upon the basin 14 the container 10 becomes fluid tight such that the solution 18 cannot easily spill out.

A soaking indicator system 22 on the container 10 aids in determining whether sufficient soaking time has been provided to the instruments 12. This system 22 comprises in gross a fill sensor 24 at the fill line 20 to detect the proper depth of solution 18, a lid closure sensor 26 to detect proper closure of the lid 16, and a data logger 28 having a timing function to time the lid 16 being closed with solution 18 to the fill line 20. In simple form, the soaking indicator system 22 employs a moisture sensor for the fill sensor 24, a contact switch for the lid closure sensor 26 and a simple countdown timer for the data logger which initiates its timing upon activation of the fill sensor 24 and lid closure sensor 26 and then provides an indication at the end of a predetermined soaking time such as by lighting an LED 27. For instance a red LED could indicate that the cycle is not yet complete and a greed LED could indicate that the cycle is complete, preferably with labels for each LED. Of course, more sophisticated systems may also be employed.

One important feature of the lid 16 is a screen 30 which is disposed below the lid 16 and sits at or below the fill line 20 when the lid 16 is closed. The screen 30 insures that buoyant instruments 12 will not float up above the level of the solution 18 and have certain portions of themselves avoid proper soaking. Preferably, it is supported on standoffs 32. For economy and construction, one of the standoffs 32 can be adapted to engage the lid closure sensor 26. Further, the standoffs 32 may bear a seal 34, as for instance silicone, which bears against the container 16 and helps to maintain leaktight configuration when the lid 16 is closed. The seal 34 could be located on other locations of the lid 16 or where the lid 16 contacts the basin 14. One or more latches, not shown, may be provided for holding the lid 16 closed.

A more sophisticated data logger 40 is shown in FIG. 2. The data logger 40 mounts on the wall of the soaking container 10 and incorporates the fill sensor 22 and the lid closure sensor 26 each of which are connected in series to a controller 42.

The controller 42 receives a high input signal only when both the lid closure 26 and liquid fill 22 sensors are closed. A high input signal to the controller 42 starts a soaking timer within the controller 42. Preferably the status of the sensors 22 and 26 and other information such as the soaking duration are displayed on a display 44, such as an LED or LCD display. The display 44 can also include a count down timer output showing the time remaining until a full soaking has been completed. Preferably it further provides some indication at to whether there is enough liquid and whether the lid is closed properly. During soaking time if either the lid is opened or the liquid level falls below the minimum fill line, the timer will reset. Satisfactory completion of a soaking cycle is displayed on the display 44.

Further, information regarding the soaking cycle is transferred to a remote base station host 46 for further processing. Such information can include the soaking time, time of completion etc. Transfer of the information to the host 46 can occur in a multitude of ways, such as through a USB link 48, RF transceiver 50, RFID (not shown) or manual entry.

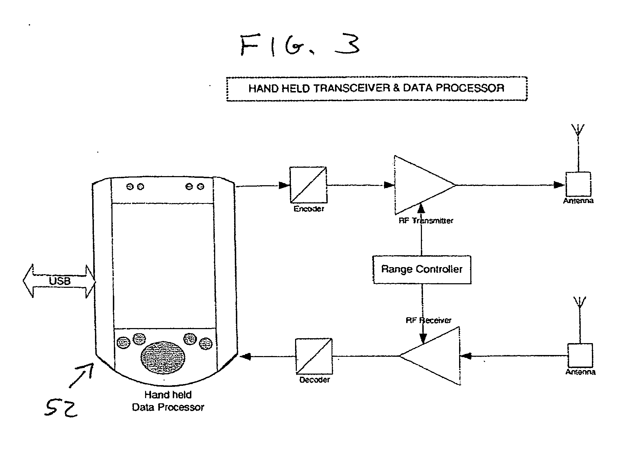

If using the RF transceiver 50 to communicate with base station and an optional hand held device 52 (FIG. 3), communication is controlled through a range controller 54 by adjusting communication frequency and/or power. The data logger controller 42 encodes and transmits data through its RF transceiver 50 using an antenna 56. Similar equipment at the base station 46 and handheld device 52 receive the signal from the antenna 56 and decode the data. Preferably, the data logger communicates with the base station 46 and hand held device 52 through high frequency and low frequency RF, respectively and the base station 46 communicates with the hand held device 52 through low frequency RF communication.

The data transferred can include a unique container identifier, soak time, user information, instrument list, fluid level, fluid type, lid status, record of container usage, instruments, record of instrument usage, and record of instrument processing methods including cleaning, disinfection or sterilization. The information can be communicated visually or electronically, locally or remotely. By viewing or retrieving the information, user can know whether the container has enough fluid, what type of fluid is in the container, whether the lid is closed properly, whether the instrument has been soaked long enough, the owner of the container, the history of the container, instrument in the container, the number of instruments, the history of the instrument, and the next processing step after soaking. The next step can be for further cleaning, decontamination, disinfection, or sterilization. The decontamination, disinfection or sterilization can be either low temperature or higher temperature process. It can also be a specific washer, decontaminator, or washer/decontaminator.

When the amount of information is small the display 44 should suffice. When more copious amounts of information are handled the handheld device 52 is preferred. It ideally communicates directly with the container's transceiver 50. Existing communication protocols such as Bluetooth or WiFi are preferred, but the invention need not be so limited.

Information about the instruments 12, such as the type, number and ID numbers thereof can be manually entered, either on a keypad or entry device on the container 10, but more preferably through the handheld device 52. A more convenient method would be to tag each instrument 12 with a machine readable tag, such as an RFID tag 60 (FIG. 1). Then, an RFID tag reader 62 incorporated into the data logger 40, or in the handheld device 52 could read and record the information automatically. Therefore, the container 10 can communicate with the user whether all previously removed instruments are properly back to in the container 10.

Preferably, the display 44, or the handheld device 52, will indicate when all of the instruments 12 are in the container 10, and if not which ones are missing. It can also display if the wrong instrument is placed into the container 10. Automated tracking of instruments in containers need not be limited to soaking containers 10, but can also be used with sterilization containers and trays used in steam or gas/plasma sterilization. In such case hardened tags up to the rigors of those environments need be employed. Especially in plasma sterilizers high emf energy can be present.

When loading a container 10 prior to a procedure a list of instruments can be downloaded to the container 10 or handheld device 52, or it may already be stored therein. Then when all the instruments are properly therein a user is provided an indication of such, and if not can be told which instruments are missing. If some of the instruments 12 are disposable, the system can be programmed to not require their return to the container 10 after a procedure.

The user can also track the location of the container 10, the use of the container 10, and the use of instruments 12 in the container. The information can be delivered to the remote host 46 for processing and storage. Optionally, the host 46 can receive container information and then send necessary information to the handheld device 52 to notify user of the container status or information.

Depending upon the types of instrument 12 or perhaps even the type of procedure in which it was used, the length of time for soaking can be modified. The type or strength of soaking solution may also be adapted. Preferably, this is automatically determined by the onboard controller 42, or by the handheld device 52 or the remote host 46 and then communicated to the onboard controller 42. If a change in soaking fluid is involved it is preferably communicated to a user by being displayed on the display 44 or on the handheld device 52.

In use, one or more instruments 12 are placed into the basin 14. Typically they are placed in the basin 14 as their use in a surgical or other medical procedure is completed. The soaking solution 18 is then placed over the instruments. The solution 18 may not be topped up to the minimal fill line 20 until all the instruments 12 are in the basin 14. When the solution 18 reaches the minimum fill line 20 the fill sensor 24 detects this and closes. After all the instruments 12 are in the basin 14 and sufficient solution 18 has been added to reach the minimum fill line 20, then the lid 16 is closed. Closure of the lid closes the lid closure sensor 26. When both sensors 24 and 26 have closed the data logger timer begins to run. After running for a predetermined time an indication of completion is provided such as by lighting the LED 27.

Different soaking solutions 18 are appropriate for use with the present invention. It may be a cleaning fluid, a disinfectant, or a sterilant. Preferably, the fluid has combined features of cleaning/disinfection or cleaning/sterilization. One well known cleaning solution is ENZOL enzymatic soaking solution available from Advanced Sterilization Products division of Ethicon, Inc. located in Irvine, CA. Alternatively, a solution of hydrogen peroxide (preferably 3 to 10% by weight, more preferably 4 to 6%) can be employed. A further option would be so employ a foam, such as a foam comprised of hydrogen peroxide, preferably incorporating a corrosion inhibitor and a lipid dissolving agent. A foam has the advantage of having less mass easing handling of the filled container and reducing the chances of spillage. A suitable foam is disclosed in co-pending U.S. Application Serial No. 11/565,126, filed November 30, 2006, the contents of which are incorporated herein by reference.

The minimum soaking time depends upon the goal sought. For soaking in the 6% peroxide foam five minutes is sufficient to dissolve dried blood, and for a 3% foam ten minutes. Such soaking times are also sufficient to inactivate most common pathogens of concern to hospital personnel.

The foam volume may decrease over time. Therefore, container with foam may have an automated triggering mechanism to regenerate foam in the container to the required level. The mechanism may be an agitator on the slopped bottom of the container. The agitator may be driven by a motor and a power source. Alternatively, air can be pumped through the foam. The triggering mechanism may be a timer or a fluid level sensor.

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims or the equivalents thereof.

## Claims

1. A system for holding and tracking medical instruments, the system comprising:
a container for holding at least one of the medical instruments associated with the container;
each of the medical instruments bearing a machine readable identification tag bearing information about the at least one instrument;
a tag reader associated with the container; and
a control system linked to the tag reader which compares the information as read by the tag reader with pre-programmed data to match the at least one instrument to the container.

2. A system according to claim 1 wherein the control system further includes an output device which indicates based upon the information read from the tag and the pre-programmed data whether the at least one instrument belongs in the container.

3. A system according to claim 1 wherein a plurality of the instruments are associated with the container and wherein the control system is programmed to detect whether all of the instruments are properly within the container.

4. A system according to claim 3 wherein the control system further includes an output device to inform a user whether all of the instruments are properly within the container.

5. A system according to claim 4 wherein when one or more of the instruments associated with the container are not in the container, the output device further lists which of the instruments are missing from the container.

6. A system according to claim 4 wherein if one or more instruments not associated with the container are detected in the container the output device further lists which instruments are improperly in the container.

7. A system according to claim 3 wherein each of the instruments is identified by its type.

8. A system according to claim 3 wherein each of the instruments is identified with a unique identifier.

9. A system according to claim 1 wherein the tags are RFID tags and the tag reader is an RFID tag reader.

10. A system according to claim 1 wherein the tag reader is located on the container.

11. A system according to claim 1 wherein the tag reader is provided on a unit separate from the container.

12. A method for holding and tracking medical instruments, the method comprising:
associating one or more of the medical instruments with a container and storing that association within a control system associated with the container, each of the medical instruments bearing a machine readable identification tag bearing information about itself;
querying a contents of the container with a tag reader to identify which instruments are therein; and
determining whether the container contains each of the instruments associated with the container and only those instruments and outputting that determination to a user.

13. A method according to claim 12 wherein a plurality of the instruments are associated with the container and the method includes the step of informing the user whether all of the instruments are properly within the container.

14. A method according to claim 13 and further including the step of when one or more of the instruments associated with the container are not in the container, informing the user which of the instruments are missing from the container.

15. A method according to claim 14 and further including the step of if one or more instruments not associated with the container are detected in the container informing the user which instruments are improperly in the container.

16. A method according to claim 12 wherein each of the one or more instruments is identified by its type.

17. A method according to claim 12 wherein each of the one or more instruments is identified with a unique identifier.

18. A method according to claim 12 wherein the tags are RFID tags and the tag reader is an RFID tag reader.

19. A method according to claim 12 wherein the instruments comprise a set designed for a medical procedure and the method includes the step of making sure each of the instruments of the set are placed into the container prior to the procedure.

20. A method according to claim 19 wherein the method further comprises the step of ensuring that each of the instruments in the set which are reusable are returned to the container after the medical procedure.
